# EUROPEAN PATENT APPLICATION

(11) **EP 4 246 427 A1**
(43) Date of publication of application: **20.09.2023**
(21) Application number: 22161888.7
(22) Date of filing: 14.03.2022
(51) Int. Cl.: G06T 3/00

(54) **PROVIDING NORMALISED MEDICAL IMAGES**

(71) Applicant: Koninklijke Philips N.V., 5656 AG Eindhoven (NL)
(72) Inventor: GOOßEN, André, Eindhoven (NL); GRAß, Michael, Eindhoven (NL); BUELOW, Thomas, Eindhoven (NL); KROENKE, Sven, Eindhoven (NL); VON BERG, Jens, Eindhoven (NL); HARDER, Tim Philipp, Eindhoven (NL); SAALBACH, Axel, Eindhoven (NL)
(74) Representative: Philips Intellectual Property & Standards

(57) **Abstract**

A computer-implemented method of providing normalised medical images (110' ₁..ₙ) representing a region of interest (120) in a subject, is provided. The method includes: warping medical images (110₁..ₙ) in a temporal series to an atlas image (130), to provide normalised medical images (110'₁..ₙ) having a warped region of interest (120'₁..ₙ) for comparison with the region of interest (120) in the atlas image (130). The method also includes outputting the normalised medical images (110'₁..ₙ), and/or outputting a magnitude of a change in the warped region of interest (120'₁..ₙ) between the normalised medical images (110'₁..ₙ).

## Description

### FIELD OF THE INVENTION

The present disclosure relates to providing normalised medical images representing a region of interest in a subject. A computer-implemented method, a computer program product, and a system, are disclosed.

### BACKGROUND OF THE INVENTION

Clinical investigations often involve the acquisition of medical images of a subject. The images may be acquired at different points in time, i.e. as a temporal series of images. The images may be used to identify temporal changes in a region of interest, and thereby assess the progression of a medical condition in the subject. However, differences in the manner in which the images are acquired can hamper clinical investigations. For example, differences in the subject's posture, differences in the viewing angle of a medical imaging system, and differences in the amount of ionising radiation dose that are used to acquire the images, exacerbate the challenge of identifying temporal changes in the region of interest itself. Such differences therefore degrade the diagnostic value of the images, and can lead to an erroneous diagnosis of a subject's condition.

Some clinical settings permit the positioning of a subject in an optimal arrangement with respect to an imaging system. In such settings there may be a relatively small amount of inter-image variability arising from differences in the manner in which the images are acquired. However, in other clinical settings, it may be impractical, or even impossible to position the subject in an optimal arrangement. For example in intensive care settings it may be impractical to acquire images of a subject with a desired posture, or with the subject in a desired position with respect to a medical imaging system.

By way of an example, the acquisition of diagnostic chest X-ray images in an intensive care setting in order to assess the progression of a lung condition, can be a challenging task for a radiographer. The immobility of the subject, who is typically bed-bound, the limited space in which to manoeuvre a mobile X-ray imaging system, and the bulky nature of such imaging systems, are just some of the confounding factors that present challenges to the radiographer. The resulting X-ray images often suffer from degraded image quality as a result of the sub-optimal field of view, sub-optimal perspective, restrictions on X-ray dose, and limitations of the subject's posture such as the subject's state of inspiration, the position of their arms, and their semi-erect position. Consequently, the X-ray images that are acquired in an intensive care setting may have significantly lower image quality than images that are acquired during standard X-ray thorax exams. This makes it challenging to interpret the X-ray images. In particular it is the fact that these confounding factors are not constant over time, and that they differ between each of the images, that makes it challenging to assess longitudinal changes in the subject's condition. As a result, a radiologist interpreting such images may instinctively compensate for such differences, which can give rise to an erroneous diagnosis of the lung condition.

Consequently, there is a need for improvements that facilitate the identification of temporal changes in medical images.

### SUMMARY OF THE INENTION

According to one aspect of the present disclosure, a computer-implemented method of providing normalised medical images representing a region of interest in a subject, is provided. The method includes:
receiving image data comprising a temporal series of 2D medical images including a region of interest in the subject;
receiving an atlas image representing the region of interest; and for each medical image in the temporal series:
   warping the medical image to the atlas image, to provide a normalised medical image having a warped region of interest for comparison with the region of interest in the atlas image; and
wherein the method further comprises outputting the normalised medical images, and/or outputting a magnitude of a change in the warped region of interest between the normalised medical images.

Since, in the above method, the medical images in the temporal series are warped to an atlas image, the shape of the region of interest is more similar in each of the normalised medical images. This facilitates a more reliable comparison between the region of interest in the images, thereby permitting a more reliable assessment of longitudinal changes in the subject's condition.

Further aspects, features, and advantages of the present disclosure will become apparent from the following description of examples, which is made with reference to the accompanying drawings.

### BRIEF DESCRIPTION OF THE DRAWINGS

Fig. 1 is a flowchart illustrating an example of a method of providing normalised medical images representing a region of interest in a subject, in accordance with some aspects of the present disclosure.
Fig. 2 is a schematic diagram illustrating an example of a system 200 for providing normalised medical images representing a region of interest in a subject, in accordance with some aspects of the present disclosure.
Fig. 3 illustrates an example of a lung field atlas image 130, in accordance with some aspects of the present disclosure.
Fig. 4 illustrates an example of a rib cage atlas image 130, in accordance with some aspects of the present disclosure.
Fig. 5 illustrates an example of the warping of two 2D medical images in a temporal series, 110₁, 110₂, to an atlas image, to provide respective normalised medical images 110'₁, 110'₂, in accordance with some aspects of the present disclosure.
Fig. 6 illustrates an example of the result of applying feature detector to a medical image 110₁ to identify a plurality of landmarks in the medical image, in accordance with some aspects of the present disclosure.
Fig. 7 illustrates an example of the warping of two 2D medical images in a temporal series, 110₁, 110₂, to an atlas image, and the adjustment of the intensity of the medical images, in accordance with some aspects of the present disclosure.
Fig. 8 illustrates an example of the warping of two 2D medical images in a temporal series, 110₁, 110₂, to an atlas image, the adjustment of the intensity of the medical images, and the suppression of image features outside a lung region of interest, in accordance with some aspects of the present disclosure.
Fig. 9 illustrates an example of a temporal series of 2D medical images 110_{1..n} (upper), a temporal sequence of corresponding normalised images 110'_{1..n} in which the temporal series of 2D medical images 110_{1..n} have been both warped to an atlas image and had their intensities adjusted to provide adjusted intensity values (middle), and a temporal sequence of corresponding normalised images in which image features outside a lung region of interest have been suppressed (lower), in accordance with some aspects of the present disclosure.

### DETAILED DESCRIPTION OF EMBODIMENTS

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer implemented method, may be implemented in a computer program product, and in a system, in a corresponding manner.

In the following description, reference is made to computer-implemented methods that involve providing normalised medical images representing a region of interest in a subject. In some examples, reference is made to the region of interest being a lung of a subject. However, it is to be appreciated that this region of interest serves only as an example, and that the methods disclosed herein may alternatively be used to provide normalised medical images representing other regions of interest in a subject.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact diskread only memory "CD-ROM", compact disk-read/write "CD-R/W", Blu-Ray^{™} and DVD.
As mentioned above, there is a need for improvements that facilitate the identification of temporal changes in medical images.

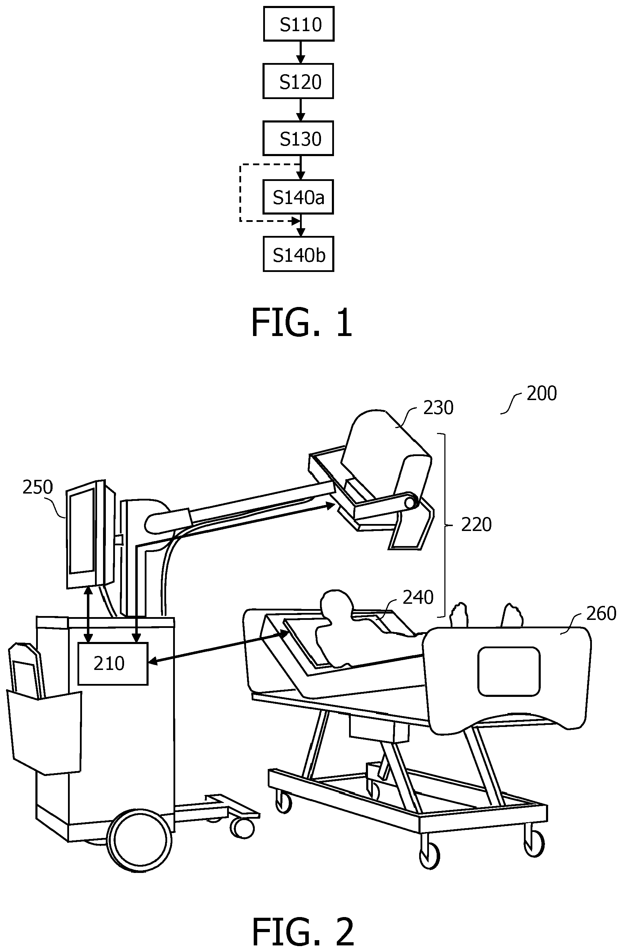

Fig. 1 is a flowchart illustrating an example of a method of providing normalised medical images representing a region of interest in a subject, in accordance with some aspects of the present disclosure. Fig. 2 is a schematic diagram illustrating an example of a system 200 for providing normalised medical images representing a region of interest in a subject, in accordance with some aspects of the present disclosure. Operations described in relation to the method illustrated in Fig. 1, may also be performed in the system 200 illustrated in Fig. 2, and vice versa. With reference to Fig. 1, the computer-implemented method of providing normalised medical images 110'_{1..n} representing a region of interest 120 in a subject, includes:
receiving S110 image data comprising a temporal series of 2D medical images 110_{1..n} including a region of interest 120 in the subject;
receiving S120 an atlas image 130 representing the region of interest 120; and for each medical image 110_{1..n} in the temporal series:
   warping S130 the medical image to the atlas image 130, to provide a normalised medical image 110'_{1..n} having a warped region of interest 120'_{1..n} for comparison with the region of interest 120 in the atlas image 130; and
wherein the method further comprises outputting S140a the normalised medical images 1 10'_{1..n}, and/or outputting S140b a magnitude of a change in the warped region of interest 120'_{1..n} between the normalised medical images 110'_{1..n}.

Since, in the above method, the medical images in the temporal series are warped to an atlas image, the shape of the region of interest is more similar in each of the normalised medical images. This facilitates a more reliable comparison between the region of interest in the images, thereby permitting a more reliable assessment of longitudinal changes in the subject's condition.

With reference to Fig. 1, in the operation S110, image data is received. The image data comprises a temporal series of 2D medical images 110_{1..n} including a region of interest 120 in the subject. The received 2D medical images form a temporal series in the sense that the images are acquired at different points in time. The images may be acquired periodically, i.e. at regular intervals, or intermittently, i.e. at irregular intervals. The temporal series of 2D medical images may be acquired over a period of time, such as over a period of minutes, hours, days, weeks, or a longer period. The temporal series of 2D medical images may represent a so-called longitudinal study on a subject. In general, the region of interest represented in the images may be any anatomical region. For instance, the region of interest may be a lung, the heart, the liver, the kidney, and so forth. By way of an example, the temporal series of 2D medical images 110_{1..n} that are received in the operation S110 may represent the chest of the subject. The temporal series of 2D medical images may for instance represent daily images of the chest that are acquired as part a longitudinal study in a subject to assess the progression of Covid 19 in the lungs of the subject.

The temporal series of 2D medical images 110_{1..n} that is received in the operation S110 may in general be generated by any 2D medical imaging system. The 2D medical images may include X-ray images, or ultrasound images, for example. The 2D X-ray images may in general be generated by any projection X-ray imaging system. Examples of current X-ray projection imaging systems that may generate the 2D medical images 110_{1..n} include the MobileDiagnost M50, which is a mobile X-ray imaging system, the DigitalDiagnost C90, which is ceiling-mounted X-ray imaging system, and the Azurion 7, which includes a source-detector arrangement mounted to a C-arm, all of which are marketed by Philips Healthcare, Best, the Netherlands. The 2D ultrasound images may be generated by any 2D ultrasound imaging system.

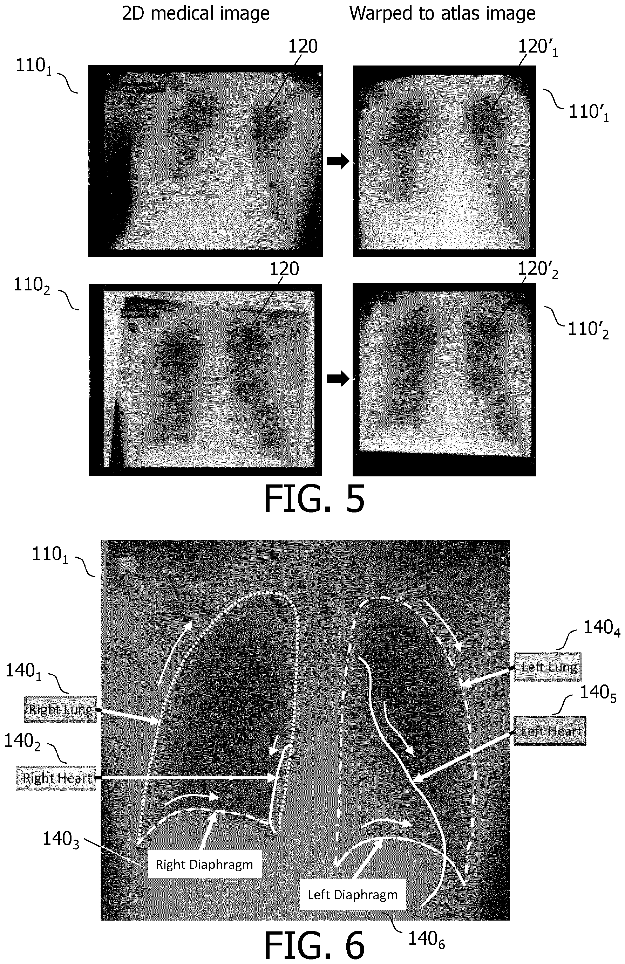

By way of an example, the 2D medical images 110_{1..n} that are received in the operation S110 may include X-ray images that are generated by the projection X-ray imaging system 220 illustrated in Fig. 2. The projection X-ray imaging system illustrated in Fig. 2 is a so-called mobile X-ray imaging system that is often used in intensive care settings in order to obtain images of subjects with restricted mobility. The projection X-ray imaging system 220 illustrated in Fig. 2 includes an X-ray source 230 and a wirelessly-coupled X-ray detector 240. The versatility of this type of X-ray imaging system facilitates the imaging of subjects in complex settings in which a subject has restricted mobility, such as the bed-bound setting illustrated in Fig. 2. However, as outlined above, it can be challenging to assess a subject's condition from images that are acquired in such settings in view of the restricted mobility of the subject, who may be unable to position themselves with an optimal posture, or even a repeatable posture, in each imaging session. An illustration of the variability in pose, and also quality, of images that may be obtained from the bed-bound setting illustrated in Fig. 2, is provided on the left-hand side of Fig. 5. Fig. 5 illustrates an example of the warping of two 2D medical images in a temporal series, 110₁, 110₂, to an atlas image, to provide respective normalised medical images 110'₁, 110'₂, in accordance with some aspects of the present disclosure. The two 2D X-ray images 110₁ and 110₂ on the left-hand side of Fig. 5 represent the chest of a subject at two different points in time. As may be appreciated, an identification of changes in the state of the subject's lungs in these images is confounded by differences in the subject's posture, and also by differences in the perspective of the X-ray imaging system with respect to the subject. It is noted that the setting illustrated in Fig. 2 serves only as an example, and it is to be appreciated that the principles disclosed herein are not limited to this type of imaging system, or to this type of setting.

Returning to Fig. 1, in general, the temporal series of 2D medical images 110_{1..n} that is received in the operation S110 may be received via any form of data communication, including for example wired, optical, and wireless communication. By way of some examples, when wired or optical communication is used, the communication may take place via signals transmitted on an electrical or optical cable, and when wireless communication is used, the communication may for example be via RF or optical signals. The temporal series of 2D medical images 110_{1..n} may be received by one or more processors, such as the one or more processors 210 illustrated in Fig. 2, for example. The one or more processors 210 may receive the 2D medical images 110_{1..n} from a 2D medical imaging system, such as the projection X-ray imaging system 220 illustrated in Fig. 2, or from a 2D ultrasound imaging system, or from another source, such as a computer readable storage medium, the Internet, or the Cloud, for example.

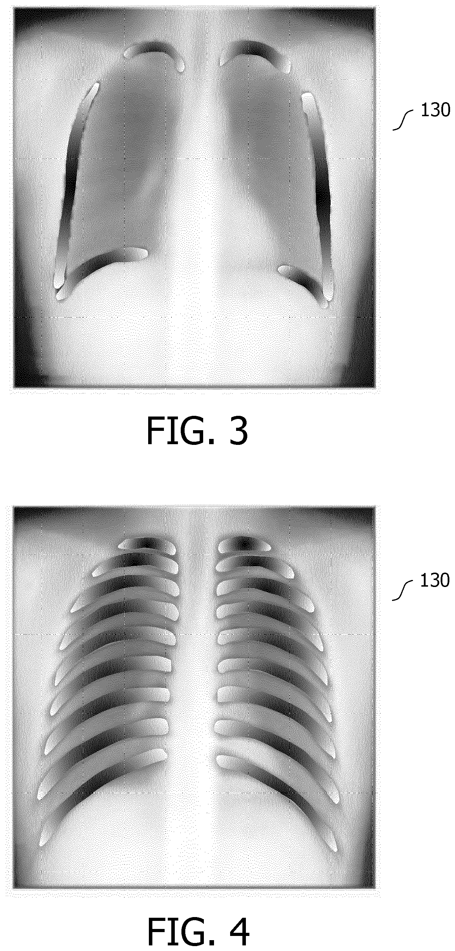

With continued reference to Fig. 1, in the operation S120 an atlas image 130 representing the region of interest 120, is received. Fig. 3 illustrates an example of a lung field atlas image 130, in accordance with some aspects of the present disclosure. Fig. 4 illustrates an example of a rib cage atlas image 130, in accordance with some aspects of the present disclosure. The atlas images 130 illustrated in Fig. 3 and Fig. 4 indicate by way of their intensity values, a probability of a structure belonging to a lung border, and to the ribs, respectively. The atlas image 130 may be received by the one or more processors 210 illustrated in Fig. 2, for example. The atlas image may be received from a database of atlas images. The atlas image may be selected from the database based on similarities between the subject, and a reference subject represented in the atlas image. For example, the atlas image may be selected based on similarities between age, gender, and size of the subject and the reference subject. The atlas image serves as a reference image of the region of interest. The atlas image may represent a preferred perspective of the region of interest in the reference subject. The atlas image may be acquired whilst the reference subject maintains a preferred posture. By way of an example, if the region of interest is the lungs, an atlas image for the lungs might be a so-called posteroanterior "PA" chest view that represents the lungs, bony thoracic cavity, mediastinum and great vessels. Such an atlas image may be acquired with the subject erect and facing an upright X-ray detector, at a specified state of inspiration, with the superior aspect of the X-ray detector a specified distance above the shoulder joints, with the chin is raised as to be out of the image field, with the shoulders rotated anteriorly to allow the scapulae to move laterally off the lung fields, and under specified operating settings (e.g. X-ray kVp energy, and exposure time) of the X-ray imaging system.

Returning to Fig. 1, in the operation S130, the medical images 110_{1..n} in the temporal series that are received in the operation S110, are then warped to the atlas image. The warping operation may be performed using various known transforms. One example of a suitable transformation is an affine transform. Other examples of suitable transformations include B-splines, thin-plate splines, and radial basis functions. The warping operation S130 may be performed based on a mapping between a plurality of corresponding landmarks 140_{1..k} represented in both the medical image and the atlas image 130. The warping operation S130 may be performed using a neural network. If the warping is performed by a neural network, the neural network may or may not explicitly use such landmarks. The landmarks 140_{1..k} may be provided by anatomical features, or by fiducial markers. By way of some examples, anatomical features such as bones, e.g. ribs, the scapula, and so forth, or organ contours, e.g. a contour of the lung, the diaphragm, the heart shadow, and so forth, may serve as anatomical landmarks. Such features are identifiable in the 2D medical images by virtue of their X-ray attenuation. Fiducial markers that are formed from X-ray attenuating materials are also identifiable in the medical images and these may also serve as landmarks. The fiducial landmarks may be located superficially, or within the body, and at known reference positions. In the latter case, the fiducial markers may have been implanted for use as a surgical guide for example. An implanted device such as a pacemaker may also serve as a fiducial marker. Fiducial markers may also be provided by interventional devices that are inserted within the body.

The landmarks 140_{1..k} in the temporal series of 2D medical images 110_{1..n}, and the corresponding landmarks in the atlas image, may be identified using various techniques. In one example, a feature detector is used to identify anatomical landmarks in the temporal series of 2D medical images 110_{1..n}. The identified landmarks are then mapped to corresponding anatomical landmarks that are labelled in the atlas image. In this example, the atlas image 130 includes a plurality of labelled anatomical landmarks; and the method described with reference to Fig. 1 also includes:
applying a feature detector to each medical image 110_{1..n} in the temporal series to identify a plurality of landmarks in the medical image that correspond to the labelled anatomical landmarks; and
wherein the mapping is determined using the identified corresponding landmarks.

In this example, the operation of applying a feature detector to the medical images 110_{1..n} in the temporal series, may be performed using an edge detector, or a model-based segmentation, or a neural network, for example. By way of an example, Fig. 6 illustrates an example of the result of applying feature detector to a medical image 110₁ to identify a plurality of landmarks in the medical image, in accordance with some aspects of the present disclosure. The 2D medical image 110₁ illustrated in Fig. 6 represents the chest, and includes bone regions such as the ribs and the spine, and an outline of the heart shadow and the lungs. In this example, the region of interest is the lung, and the feature detector has identified in the 2D medical image 110₁, outlines representing the right lung, the right side of the heart shadow, the right side of the diaphragm, the left lung, the left side of the heart shadow, and the left side of the diaphragm as landmarks 140_{1..6} respectively. These landmarks correspond to landmarks in an atlas image, such as the lung field atlas image illustrated in Fig. 3. The landmarks 140_{1..6} that have been identified in the 2D medical image 110₁, are then mapped to their corresponding landmarks in the atlas image, in order to warp the medical image 110₁ to the atlas image in the operation S130. As illustrated in Fig. 6, one or more of the landmarks that are identified and used in the mapping operation may represent the region of interest, which in this example is the lung.

The result of the warping operation S130 is to provide for each of the medical images 110_{1..n} in the temporal series, a normalised medical image 1 10'_{1..n} having a warped region of interest 120'_{1..n} for comparison with the region of interest 120 in the atlas image 130. The normalised medical images 110'_{1..n} have a warped region of interest 120'_{1..n} for comparison with the region of interest 120 in the atlas image 130 in the sense that shape differences between the warped region of interest 120'_{1..n} in each normalised medical image 1 10'_{1..n} and the region of interest 120 in the atlas image 130, have been are reduced. Since the warped regions of interest 120'₁ and 120'₂ are suitable for comparison with the region of interest 120 in the atlas image 130, they may consequently be compared with one another. The warping operation S130 thus reduces differences between the shapes of regions of interest 120'₁ and 120'2, which in turn permits a more reliable identification of temporal changes in the region of interest arising from changes in the subject's condition. In other words, the warping operation S130 provides that the warped regions of interest 120'_{1..n} in the normalised medical image 1 10'_{1..n} have a similar shape. An example of normalised medical images 1 10'_{1..n} that are provided in this manner is illustrated in the images on the right-hand side of Fig. 5. As may be appreciated, the lung regions of interest 120'₁ and 120'₂ in these images have more-similar shapes in the normalised medical images 110'₁ and 110'₂, and this permits a more reliable identification of temporal changes in the region of interest due to the progression of a lung condition.

Returning to Fig. 1, in the operations S140a, and S140b, the method performs an operation of outputting S140a the normalised medical images 110'_{1..n}, and/or an operation of outputting S140b a magnitude of a change in the warped region of interest 120'_{1..n} between the normalised medical images 110'_{1..n}. The outputting of the normalised medical images 110'_{1..n} in the operation S140a may be performed in various ways, including displaying, printing, and storing the normalised medical images 110'_{1..n}. The images may be displayed on a display device, such as the monitor 250 illustrated in Fig. 5, for example. By way of an example, the normalised medical images 110'_{1..n} may be displayed side-by side in the manner illustrated in Fig. 5, or as a temporal sequence, or as an overlay image, or in another manner.

The outputting of a magnitude of a change in the warped region of interest in the operation S140b may also be performed in various ways, including displaying, printing, and storing the magnitude of the change. In general, the change may represent a change in intensity, or a change in shape, of the region of interest between any two of the normalised medical images 1 10'_{1..n}. The change may be determined between consecutive images, for instance. In some examples, a statistical analysis may be applied to the warped regions of interest in order to determine the magnitude of the change. By way of some examples, if the region of interest is the lung, then the magnitude of the change may quantify a volume of a pneumothorax, i.e. a volume of a "collapsed lung", or an amount of pleural effusion, i.e. an amount of "fluid around the lung", or an amount of pulmonary edema, i.e. an "amount of fluid in the lung", or a stage of pneumonia. Such changes may be calculated by determining temporal changes in the intensity values within the lung in the normalised medical images 110'_{1..n}, or by comparing the image intensities to healthy reference values in the lung in the atlas image. In one example, the changes may be determined by e.g. contouring the lung in the normalised medical images 110'_{1..n}, assigning pixels within the lung to air or to water based on their intensity values, and estimating a volume of the respective air and water regions for each of the normalised medical images 110'₁.

By way of some examples, the outputting of the magnitude of a change in the warped region of interest in the operation S140b may include outputting a numerical value of the magnitude, e.g. as a percentage, or outputting the magnitude of the change graphically. In one example, the magnitude of the change may be represented graphically as an overlay on one or more of the normalised medical images 110'_{1..n} by representing an increase in lung volume between consecutive images in green, and a decrease in lung volume between consecutive images in red.

By outputting the normalised medical images in the operation S140a and/or outputting the magnitude of a change in the warped region of interest in the operation S140b, the method facilitates a reviewing clinician to make a more reliable comparison between the region of interest in the images. This, in turn, permits the clinician to make a more accurate assessment of longitudinal changes in the subject's condition.

Various additional operations may also be performed in accordance with the method described above with reference to Fig. 1.

In some examples, the method described with reference to Fig. 1 also includes:
adjusting an intensity of each medical image 110_{1..n} in the temporal series based on an intensity at one or more positions in the atlas image 130, or based on an intensity at one or more positions in a medical image 110_{1..n} in the temporal series, or based on an intensity at one or more positions in a reference image, to provide adjusted intensity values in the normalised medical images 110'_{1..n}; and/or
adjusting an intensity of each medical image 110_{1..n} in the temporal series using an image style transfer algorithm, to provide adjusted intensity values in the normalised medical images 110'_{1..n}.

The inventors have observed that not only is the assessment of temporal changes in medical images hampered by differences in the shape of a region of interest, between the images, but that the assessment of such changes may also be hampered by differences in the intensity scale of the images. More specifically, it is differences in the intensity scale that arise from differences in the way in which 2D medical images in a temporal series are acquired, that can hamper their comparison. By way of an example, the intensity at any point in a 2D X-ray image is dependent on the values of the X-ray energy "kVp", the exposure time, and the sensitivity of the X-ray detector that are used to acquire the image. Since the images in a temporal series are acquired over a period of time, the images in the temporal series may be generated by X-ray imaging systems that have different settings, or indeed by different X-ray imaging systems. In contrast to computed tomography images, and in which a Hounsfield unit value may be assigned to each voxel, in 2D X-ray images there is no corresponding absolute attenuation scale for the pixel intensity values. By adjusting an intensity of each medical image 110_{1..n} in the temporal series based on an intensity at one or more positions in the atlas image 130, or based on an intensity at one or more positions in a medical image 110_{1..n} in the temporal series, or based on an intensity at one or more positions in a reference image, to provide adjusted intensity values in the normalised medical images 110'_{1..n}, there is a further reduction in the inter-image variability arising from differences in the way in which the 2D medical images are acquired.

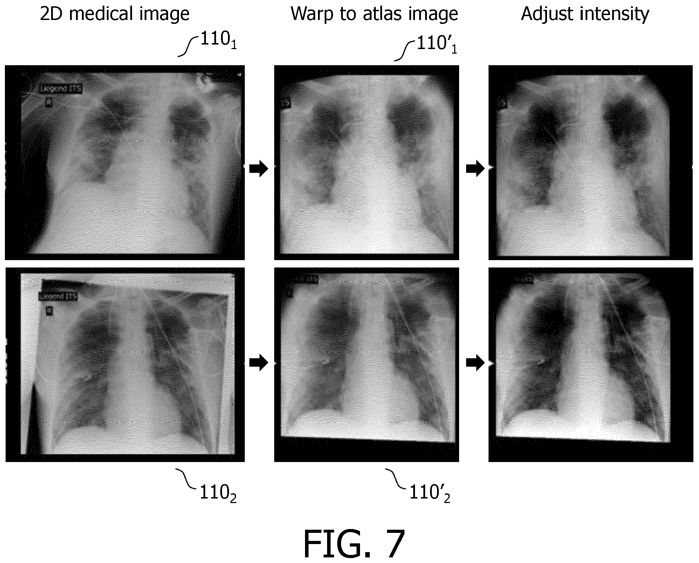

Techniques such as windowing and histogram normalisation may be used to normalise the intensity values in accordance with this example. The positions in the atlas image 130, may be defined manually, or automatically. By way of an example, if the region of interest is the lung, the positions may be defined within the lung field, or within the mediastinum, for example. The positions may be pre-defined in the atlas image, or defined based on user input that is provided via a user input device in combination with a displayed image. This example is also described with reference to Fig. 7, which illustrates an example of the warping of two 2D medical images in a temporal series, 110₁, 110₂, to an atlas image, and the adjustment of the intensity of the medical images, in accordance with some aspects of the present disclosure. The intensities in the images on the right-hand side of Fig. 7 have been adjusted in the above-described manner, and facilitate a more reliable identification of changes in the region of interest between the images.

Alternatively, or additionally, an image style transfer algorithm may be used to provide the adjusted intensity values in the normalised medical images 110'_{1..n}. Various image style transfer transforms are known for this purpose. These may be based on e.g. a Laplace pyramid, or a (convolutional) neural network, for example. An example of an image style transfer algorithm is disclosed in the document WO 2013/042018 A1. This document discloses a technique for transforming a slave image that involves generating a color or grey scale transformation based on a master image and a slave image. The transformation is used to optically adapt the slave image to the master image. This technique may be used to adjust an intensity of the medical images 110_{1..n} in the temporal series such that the adjusted intensity values in the normalised medical images 110'_{1..n} have a similar appearance, or style. The technique disclosed in this document may be applied by using the medical images 110_{1..n} in the temporal series as the slave images, and using the atlas image, or one of the medical images 110_{1..n} in the temporal series, or another reference image as the master image.

In a related example, the operation of adjusting an intensity of each medical image 110_{1..n} in the temporal series based on an intensity at one or more positions in the atlas image 130, may include:
calculating an average intensity value within a portion of the atlas image 130; and
normalising the intensity values in each medical image 110_{1..n} in the temporal series using the average intensity value.

In this example, the portion of the atlas image 130 that is used to normalise the intensity values in each medical image 110_{1..n} may be selected as a portion of the atlas image in which the image intensity is expected to be temporally-invariant over a duration of the temporal series of 2D medical images 110_{1..n}. For instance, if the region of interest is a lung, the selected portion of the atlas image may correspond to a portion of the spine since the attenuation of the spine would not be expected to change over time. Alternatively, if the region of interest is a lung, the selected portion may correspond to a portion of the lung in the atlas image that is expected to be free of disease in the temporal series of 2D medical images 110_{1..n}. For instance, if the subject's condition under investigation is pulmonary edema, then a collection of fluid over time may be expected to result in temporal changes in the image intensity in the lower portion of the lung, whereas the upper portion of the lung may be expected to remain free of disease and consequently to have a stable image intensity. In this case, the upper portion of the lung in the atlas image 130 may be used as the portion of the atlas image 130 that is used to normalise the intensity values in each medical image 110_{1..n}.

By way of an example in which the region of interest is the lung, these operations may be carried out by identifying a portion of the lung in the atlas image. The portion of the lung may be identified automatically, or based on user input received via a user input device in combination with a display device. The portion of the atlas image may represent a combination of air in the lung, and ribs surrounding the lung. An average intensity of the pixel values in this portion of the atlas image may then be calculated. This average value may then be used to normalise the intensity values of the medical images 110_{1..n} in the temporal series. With reference to Fig. 5, this may for instance include identifying a disease-free corresponding portion of the lung, e.g. towards the top of the lung in the medical images 110₁, calculating an average value of the intensity values in the corresponding disease-free portion, calculating a scaling factor as a ratio of the average intensity values in the disease-free region of the medical image 110₁ to the average intensity values in the portion of the lung in the atlas image, and scaling the image intensity values in the medical image 110₁ using the scaling factor to provide adjusted intensity values in the normalised medical image 110'₁. These operations are then be repeated for the other medical images 110_{1..n} in the temporal series. Since the portion of the lung in the atlas image is used to normalise each medical image 110_{1..n} in the temporal series, the medical images are scaled to a common reference intensity, which facilitates a more reliable comparison between the image intensities in other portions of the images.

Additional portions of the atlas image may also be used to perform the intensity normalisation described above. For example, a second region of interest may be identified within the mediastinum, or within a bone region, of the atlas image, and also used to perform the intensity normalisation. The use of two regions of interest having different attenuation values to normalise the intensity in the medical images 110_{1..n} in the temporal series may facilitate a more accurate mapping of the intensity values, and consequently a more reliable identification of changes in the regions of interest in the images.

In this example, a value of a quality metric may also be calculated and outputted for the adjusted intensity values in the normalised medical images 110'_{1..n}. The value of the quality metric may be calculated based on e.g. the statistics of the image intensity values in the portion of the atlas image, or the corresponding portion of the medical image 110'_{1..n}, or based on the quality of the mapping between the landmarks in the operation S130. The value of the quality metric may be relatively lower if the variance of the image intensity values in the portion of the atlas image is high, and vice versa. Outputting the value of the quality metric re-assures a reviewing clinician of the accuracy of the method.

The inventors have also observed that subsequent to the warping operation S130, some regions of the normalised medical images 110'_{1..n} that are outside of the region of interest may be warped to an unnatural shape. This can be confusing to a reviewing physician. In another example, the method described with reference to Fig. 1 may also include:
suppressing one or more image features outside the region of interest 120'_{1..n} in the normalised medical images 110'_{1..n}.

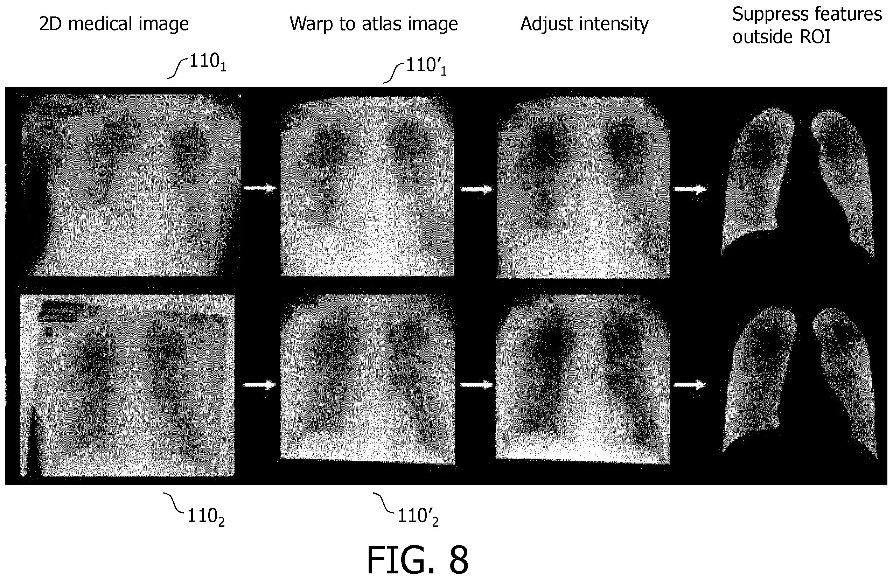

By suppressing the one or more image features outside the region of interest 120'_{1..n}, it is avoided that a reviewing clinician is presented with confounding information. This example is described with reference to Fig. 8, which illustrates an example of the warping of two 2D medical images in a temporal series, 110₁, 110₂, to an atlas image, the adjustment of the intensity of the medical images, and the suppression of image features outside a lung region of interest, in accordance with some aspects of the present disclosure. In the images on the right-hand side of Fig. 8, portions of the normalised medical images 1 10'_{1..n} outside a lung region of interest, have been suppressed. In the Fig. 8 example, the image features outside the lung region of interest have been suppressed by mapping their pixel values to a black-level pixel intensity value. However, these features may alternatively be suppressed in a different manner. For example, their pixel values may be mapped to a different pixel intensity value than a black-level pixel intensity value, or their pixel values may be adjusted gradually to a predetermined value in order to "feather-out" the region of interest. This operation of suppressing the image features outside the region of interest may be performed using the atlas image. For instance, in this example, the method described with reference to Fig. 1 may also include:
identifying the region of interest 120 in the atlas image 130;
mapping an outline of the region of interest 120 from the atlas image 130 to each of the normalised medical images 110'_{1..n}; and
adjusting image intensity values in the normalised medical images 110'_{1..n} outside the mapped outline to suppress the one or more image features outside the region of interest 120'_{1..n}.

In this example, various example regions of interest may be pre-defined in the atlas image and used to suppress the one or more image features outside the region of interest 120'_{1..n}. Alternatively, the regions of interest may be defined by segmenting the atlas image. Since in this example the outline from the atlas image is used to suppress the one or more image features outside the region of interest 120'_{1..n}, a reliable outline is used. The outline is reliable because in the operation S130 the normalised medical images 110'_{1..n} have been warped to the atlas image using the landmarks in the atlas image, and consequently the shape of the region of interest in the normalised images is similar to that in the atlas image.

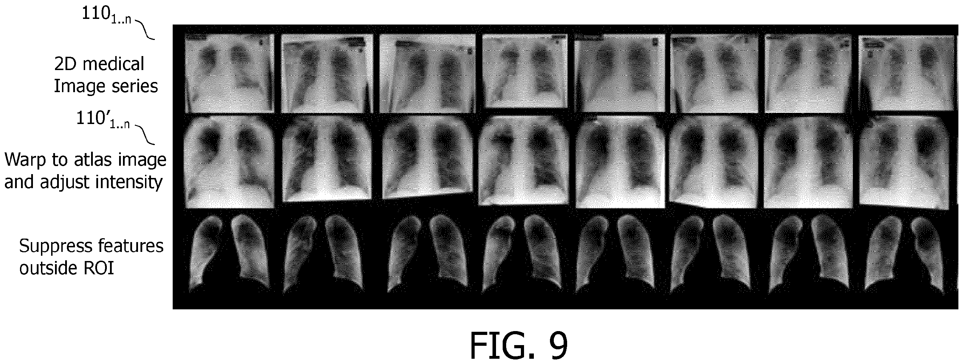

By way of a further example, Fig. 9 illustrates an example of a temporal series of 2D medical images 110_{1..n} (upper), a temporal sequence of corresponding normalised images 110'_{1..n} in which the temporal series of 2D medical images 110_{1..n} have been both warped to an atlas image and had their intensities adjusted to provide adjusted intensity values (middle), and a temporal sequence of corresponding normalised images in which image features outside a lung region of interest have been suppressed (lower), in accordance with some aspects of the present disclosure. Fig. 9 illustrates the aforementioned operations for more images than the two images illustrated in Fig. 5, Fig. 7 and Fig. 8, and is a further illustration of the amount of variability that can be observed in 2D medical images 110_{1..n} from a longitudinal study on a subject, and the advantages of the normalised medical images 110'_{1..n} that are provided by the method described herein.

In another example, the method described with reference to Fig. 1 may also include:
performing a statistical analysis of image intensity values within the region of interest 120'_{1..n} in the normalised medical image 110'_{1..n}; and
wherein the outputting S140b a magnitude of a change in the warped region of interest 120'_{1..n} between the normalised medical images 110'_{1..n}, comprises:
   outputting the result of the statistical analysis for the normalised medical images 110'_{1..n}.

Various statistical analysis methods may be applied to the image intensity values within the region of interest 120'_{1..n} in the normalised medical image 110'_{1..n}. One example implementation of such a statistical analysis is based on a local patch-based analysis of the pixel-values in the normalised medical images 110'_{1..n}. In this example, statistics may be generated for a patch in each of the normalised medical images 110'_{1..n}, _{1..n}. This may include determining the mean and standard deviation of the pixel values within the patch, assuming e.g. a Gaussian distribution. When comparing corresponding patches of e.g. two images A and B in the normalised medical images 110'_{1..n}, the Gaussian statistics of the patch of image A may be used to estimate how likely the measured intensity values in the patch from image B is. This likelihood may be thresholded in order to provide a prediction for a change within the patch. The likelihood may also be visualized as an overlay of images A and B in order permit a reviewing clinician to analyse the detected changes in the images over time. This visualization may consequently permit the reviewing clinician to assess whether the detected changes are statistically relevant, or e.g. whether they only stem from an imperfect normalization procedure.

In a related example, the operation of outputting the result of the statistical analysis for the normalised medical images 1 10'_{1..n} may include displaying a spatial map of the statistical analysis as an overlay on one or more of the normalised medical images 110'_{1..n}. Providing the result of the statistical analysis in this manner may facilitate the clinician to provide an efficient analysis of the progression of a medical condition in the subject.

In another example, a computer program product is provided. The computer program product comprises instructions which when executed by one or more processors, cause the one or more processors to carry out a method of providing normalised medical images 1 10'_{1..n} representing a region of interest 120 in a subject. The method comprises:
receiving S110 image data comprising a temporal series of 2D medical images 110_{1..n} including a region of interest 120 in the subject;
receiving S120 an atlas image 130 representing the region of interest 120; and
for each medical image in the temporal series:
   warping S130 the medical image to the atlas image 130, to provide a normalised medical image 110'_{1..n} having a warped region of interest 120'_{1..n} for comparison with the region of interest 120 in the atlas image 130; and
   wherein the method further comprises outputting S140a the normalised medical images 110'_{1..n}, and/or outputting S140b a magnitude of a change in the warped region of interest 120'_{1..n} between the normalised medical images 110'_{1..n}.

In another example, a system 200 for providing normalised medical images 110'_{1..n} representing a region of interest 120 in a subject, is provided. The system comprises one or more processors 210 configured to:
receive S110 image data comprising a temporal series of 2D medical images 110_{1..n} including a region of interest 120 in the subject;
receive S120 an atlas image 130 representing the region of interest 120; and for each medical image in the temporal series:
   warp S130 the medical image to the atlas image 130, to provide a normalised medical image 110'_{1..n} having a warped region of interest 120'_{1..n} for comparison with the region of interest 120 in the atlas image 130; and
wherein the one or more processors are further configured to output S 140a the normalised medical images 110'_{1..n}, and/or output S140b a magnitude of a change in the warped region of interest 120'_{1..n} between the normalised medical images 110'_{1..n}.
An example of the system 200 is illustrated in Fig. 2. It is noted that the system 200 may also include one or more of: a 2D medical imaging system for generating the image data that is received in the operation S110, such as for example the projection X-ray imaging system 220 illustrated in Fig. 2; a display device such as the monitor 250, a tablet, and so forth, for displaying the outputted normalised medical images 1 10'_{1..n}, and/or outputting a magnitude of a change in the warped region of interest 120'_{1..n} between the normalised medical images 110'_{1..n}, the results of a statistical analysis of image intensity values, and so forth; a patient bed 260; and a user input device (not illustrated in Fig. 2) for receiving user input in relation to operations carried-out by the system, such as a keyboard, a mouse, a touchscreen, and so forth.

The above examples are to be understood as illustrative of the present disclosure, and not restrictive. Further examples are also contemplated. For instance, the examples described in relation to computer-implemented methods, may also be provided by the computer program product, or by the computer-readable storage medium, or by the system 200, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A computer-implemented method of providing normalised medical images (110'_{1..n}) representing a region of interest (120) in a subject, the method comprising:
receiving (S110) image data comprising a temporal series of 2D medical images (110_{1..n}) including a region of interest (120) in the subject;
receiving (S120) an atlas image (130) representing the region of interest (120); and for each medical image (110_{1..n}) in the temporal series:
warping (S130) the medical image to the atlas image (130), to provide a normalised medical image (110'_{1..n}) having a warped region of interest (120'_{1..n}) for comparison with the region of interest (120) in the atlas image (130); and
wherein the method further comprises outputting (S140a) the normalised medical images (110'_{1..n}), and/or outputting (S140b) a magnitude of a change in the warped region of interest (120'_{1..n}) between the normalised medical images (110'_{1..n}).

2. The computer-implemented method according to claim 1, wherein the warping (S130) is based on a mapping between a plurality of corresponding landmarks (140_{1..k}) represented in both the medical image and the atlas image (130).

3. The computer-implemented method according to claim 1 or claim 2, wherein the atlas image (130) comprises a plurality of labelled anatomical landmarks; and
wherein the method further comprises:
applying a feature detector to each medical image (110_{1..n}) in the temporal series to identify a plurality of landmarks in the medical image that correspond to the labelled anatomical landmarks; and
wherein the mapping is determined using the identified corresponding landmarks.

4. The computer-implemented method according to claim 3, wherein the applying a feature detector is performed using an edge detector, or a model-based segmentation, or a neural network.

5. The computer-implemented method according to claim 1, wherein the method further comprises:
adjusting an intensity of each medical image (110_{1..n}) in the temporal series based on an intensity at one or more positions in the atlas image (130), or based on an intensity at one or more positions in a medical image (110_{1..n}) in the temporal series, or based on an intensity at one or more positions in a reference image, to provide adjusted intensity values in the normalised medical images (110'_{1..n});
and/or
adjusting an intensity of each medical image (110_{1..n}) in the temporal series using an image style transfer algorithm, to provide adjusted intensity values in the normalised medical images (110'_{1..n}).

6. The computer-implemented method according to claim 5, wherein the adjusting an intensity of each medical image (110_{1..n}) in the temporal series based on an intensity at one or more positions in the atlas image (130) comprises:
calculating an average intensity value within a portion of the atlas image (130); and
normalising the intensity values in each medical image (110_{1..n}) in the temporal series using the average intensity value.

7. The computer-implemented method according to claim 1, wherein the method further comprises:
suppressing one or more image features outside the region of interest (120'_{1..n}) in the normalised medical images (110'_{1..n}).

8. The computer-implemented method according to claim 7, wherein the method further comprises:
identifying the region of interest (120) in the atlas image (130);
mapping an outline of the region of interest (120) from the atlas image (130) to each of the normalised medical images (110'_{1..n}); and
adjusting image intensity values in the normalised medical images (110'_{1..n}) outside the mapped outline to suppress the one or more image features outside the region of interest (120'_{1..n}).

9. The computer-implemented method according to any previous claim, wherein the method further comprises:
performing a statistical analysis of image intensity values within the region of interest (120'_{1..n}) in the normalised medical image (110'_{1..n}); and
wherein the outputting (S140b) a magnitude of a change in the warped region of interest (120'_{1..n}) between the normalised medical images (110'_{1..n}), comprises:
outputting the result of the statistical analysis for the normalised medical images (110'_{1..n}).

10. The computer-implemented method according to claim 9, wherein the outputting the result of the statistical analysis for the normalised medical images (110'_{1..n}) comprises:
displaying a spatial map of the statistical analysis as an overlay on one or more of the normalised medical images (110'_{1..n}).

11. The computer-implemented method according to any previous claim, wherein the temporal series of 2D medical images (110_{1..n}) comprise X-ray images or ultrasound images.

12. The computer-implemented method according to any previous claim, wherein the temporal series of 2D medical images (110_{1..n}) comprise X-ray images generated by a mobile X-ray imaging system.

13. The computer-implemented method according to any previous claim, wherein the temporal series of 2D medical images (110_{1..n}) represent a longitudinal study on the subject.

14. A computer program product comprising instructions which when executed by one or more processors, cause the one or more processors to carry out a method of providing normalised medical images (110'_{1..n}) representing a region of interest (120) in a subject, the method comprising:
receiving (S110) image data comprising a temporal series of 2D medical images (110_{1..n}) including a region of interest (120) in the subject;
receiving (S120) an atlas image (130) representing the region of interest (120); and for each medical image in the temporal series:
warping (S130) the medical image to the atlas image (130) to provide a normalised medical image (110'_{1..n}) having a warped region of interest (120'_{1..n}) for comparison with the region of interest (120) in the atlas image (130); and
wherein the method further comprises outputting (S140a) the normalised medical images (110'_{1..n}), and/or outputting (S140b) a magnitude of a change in the warped region of interest (120'_{1..n}) between the normalised medical images (110'_{1..n}).

15. A system (200) for providing normalised medical images (110'_{1..n}) representing a region of interest (120) in a subject, the system comprising one or more processors (210) configured to:
receive (S110) image data comprising a temporal series of 2D medical images (110_{1..n}) including a region of interest (120) in the subject;
receive (S120) an atlas image (130) representing the region of interest (120); and
for each medical image in the temporal series:
warp (S130) the medical image to the atlas image (130) to provide a normalised medical image (110'_{1..n}) having a warped region of interest (120'_{1..n}) for comparison with the region of interest (120) in the atlas image (130); and
wherein the one or more processors are further configured to output (S 140a) the normalised medical images (110'_{1..n}), and/or output (S140b) a magnitude of a change in the warped region of interest (120'_{1..n}) between the normalised medical images (110'_{1..n}).
